(19) Europäisches Patentamt
European Patent Office
Office européen des brevets

(11) Publication number: **0 005 055 A2**

(12) **EUROPEAN PATENT APPLICATION**

(21) Application number: 79300645.3

(22) Date of filing: 19.04.79

(51) Int. Cl.²: C 07 D 337/12
A 61 K 31/38
//C07C79/46, C07C149/40

(30) Priority: 21.04.78 US 898602

(43) Date of publication of application: 31.10.79 Bulletin 79/22

(84) Designated Contracting States: BE CH DE FR GB NL SE

(71) Applicant: SYNTEX (U.S.A.) INC.
3401 Hillview Avenue
Palo Alto, California 94304(US)

(72) Inventor: Ackrell, Jack
192 Webster
Palo Alto California 94301(US)

(74) Representative: Armitage, Ian Michael et al,
MEWBURN ELLIS & CO. 70/72 Chancery Lane
London WC2A 1AD(GB)

(54) **6,11-Dihydrodibenzo-thiepin-11-ones and their S-oxides, methods for their preparation and pharmaceutical compositions containing them.**

(57) Novel 6, 11-dihydrodibenzo-[b.e.]-thiepin-11-one S-oxides, and intermediates useful in the preparation thereof, having the formulae

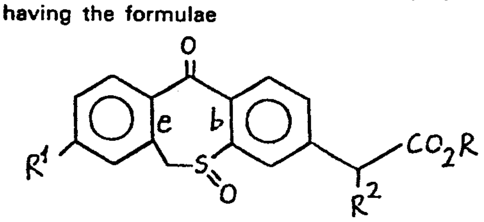

(I)

and

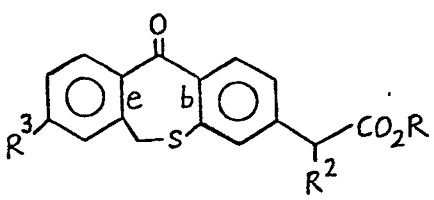

(II)

wherein R is hydrogen, an alkyl group containing from 1 to twelve carbon atoms or a pharmaceutically acceptable salt thereof when R is hydrogen, $R^1$ is hydrogen, chloro or methoxy, $R^2$ is hydrogen or methyl, and $R^3$ is chloro or methoxy.

The free acid or ester of compound II, or its 8-$R^3$ unsubstituted equivalent, can be converted to the corresponding compound I by oxidation. The compounds and compositions containing them can be used for treating inflammation or other conditions in mammals.

EP 0 005 055 A2

Croydon Printing Company Ltd.

TITLE MODIFIED see front page



## NOVEL 6, 11-DIHYDRODIBENZO-THIEPIN-11-ONE S-OXIDES, METHODS OF PREPARATION, COMPOSITIONS AND USES THEREOF AND INTERMEDIATES USEFUL IN THE PREPARATION THEREOF

This invention relates to pharmaceutically useful 6, 11-dihydrodibenzo-[b.e.]-thiepin-11-ones.

Japanese Patent Publication No. 425/72, published January 7, 1972, broadly discloses compounds of the formula:

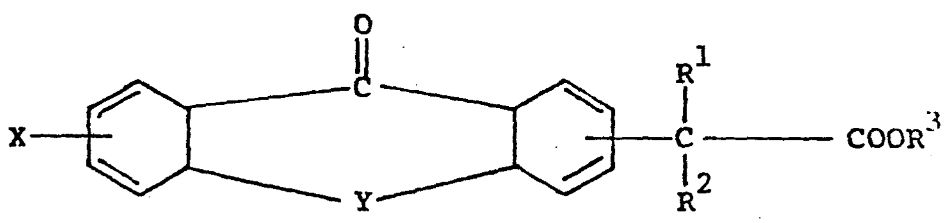

wherein $R^1$ , $R^2$ and $R^3$ are each a hydrogen atom or a lower alkyl group (e.g. methyl, ethyl, propyl and isopropyl); X is a hydrogen atom, a halogen atom (Cl, Br, I and F) or a lower alkyl group; and Y is an oxygen atom, $-CH_2S-$, or 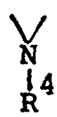 (wherein $R^4$ is a hydrogen atom or a lower alkyl group) or the salt thereof; and specifically names in Example 3 thereof 11-oxo-6,11-dihydro-dibenzo [b.e.] thiepin-2-yl-acetic acid.

German OLS 24 42 060, laid open May 7, 1975, broadly discloses oxepin compound of the formula:

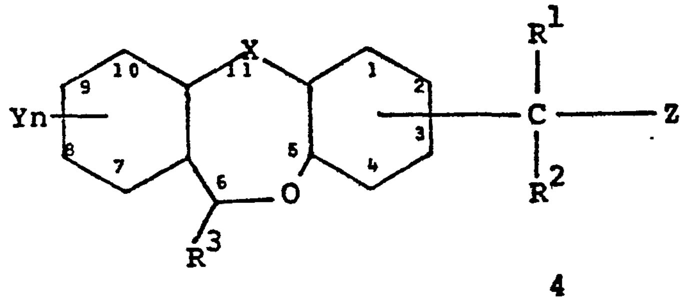

wherein X is C=O, CHCl, CHBr, $CH_2$, or $CHOR^4$; Y is alkyl of from 1 to 4 carbon atoms, alkoxy of from 1 to 4 carbon atoms, halogen or trifluoromethyl; n is an integer O, 1, 2 or 3; Z is $COOR^5$, $CH_2OR^5$, $CONR^5_2$ or $CONHOR^5$; and $R^1$ to $R^5$ are hydrogen, or alkyl of from 1 to 4 carbon atoms.

Certain oxepin compounds are also disclosed in Belgian Patent No. 818,055, laid open November 18, 1974.

Compounds within the formula

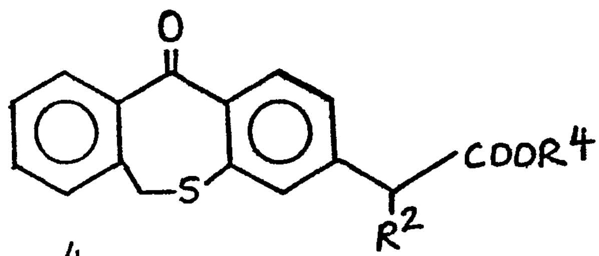

where $R^2$ and $R^4$ are defined hereinafter, are described in U.S. Patents Nos. 4000288 and 4000308. However it has now been found, as further described hereinafter, that the corresponding S-oxides exhibit certain improved properties compared with the non-S-oxides.

The compounds of the present invention have the formulae:

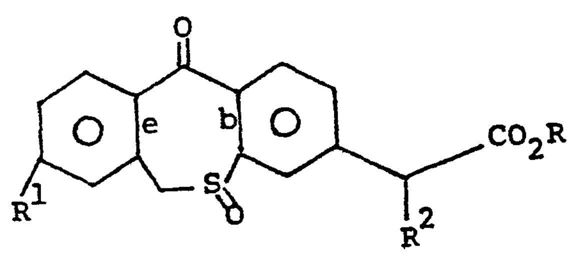

(I)

and

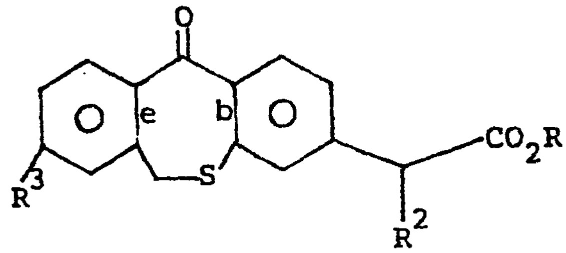

(II)

wherein R is hydrogen, an alkyl group containing from one to twelve carbon atoms or a pharmaceutically acceptable salt thereof when R is hydrogen, $R^1$ is hydrogen, chloro or methoxy, $R^2$ is hydrogen or methyl, and $R^3$ is chloro or methoxy.

Also included in this invention are compositions and methods of use for the compounds Formulas (I) and (II).

The term "alkyl" refers to and includes branched and straight chain hydrocarbons containing from one to twelve carbon atoms. Typical alkyl groups include methyl, ethyl, propyl, isopropyl, butyl, tertiary butyl, isoamyl, neopentyl, isopentyl, hexyl, octyl, nonyl, isodecyl, 6-methyldecyl, dodecyl, and the like.

The term "pharmaceutically acceptable salts" refers to salts prepared from pharmaceutically acceptable non-toxic bases including inorganic bases and organic bases. Salts derived from inorganic bases include sodium, potassium, lithium, ammonia, calcium, magnesium, ferrous, zinc, copper, manganous, aluminum, ferric, manganic salts and the like. Particularly preferred are the ammonia, potassium, sodium, calcium and magnesium salts. Salts derived from pharmaceutically acceptable organic non-toxic bases include salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, 2-dimethylaminoethanol, 2-diethylaminoethanol, tromethamine, lysine, arginine, histidine, caffeine, procaine, hydrabamine, choline, betaine, ethylenediamine, glucosamine, methylglucamine, theobromine, purines, piperazine, piperidine, N-ethylpiperidine, polyamine resins and the like. Particularly preferred organic non-toxic bases are isopropylamine, diethylamine, ethanolamine, piperidine, tromethamine, choline and caffeine.

Some of novel compounds of this invention, the compounds of Formula (II), are prepared according to the reaction scheme which follows:

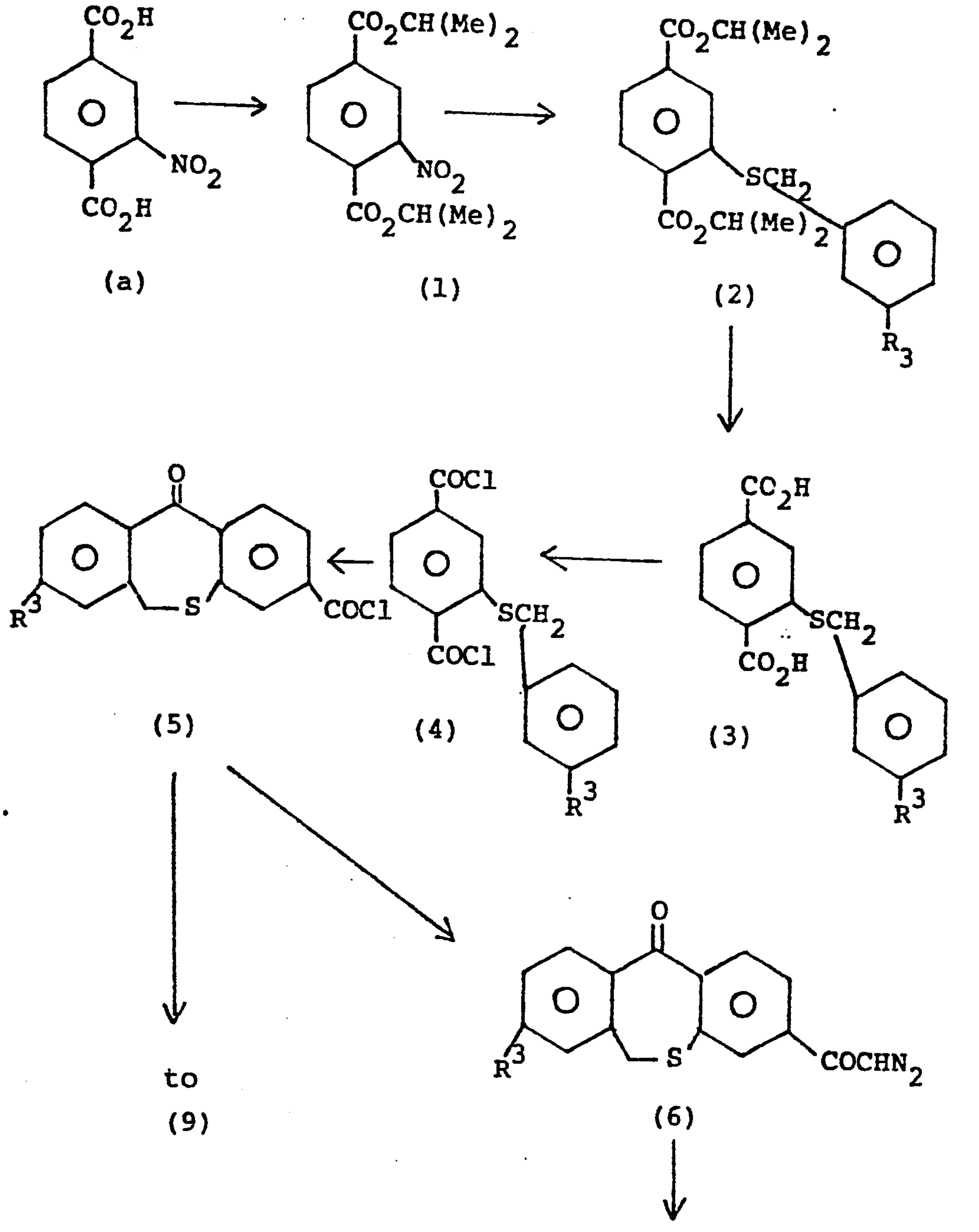


(a) (1) (2)

(5) (4) (3)

(9)

(6)

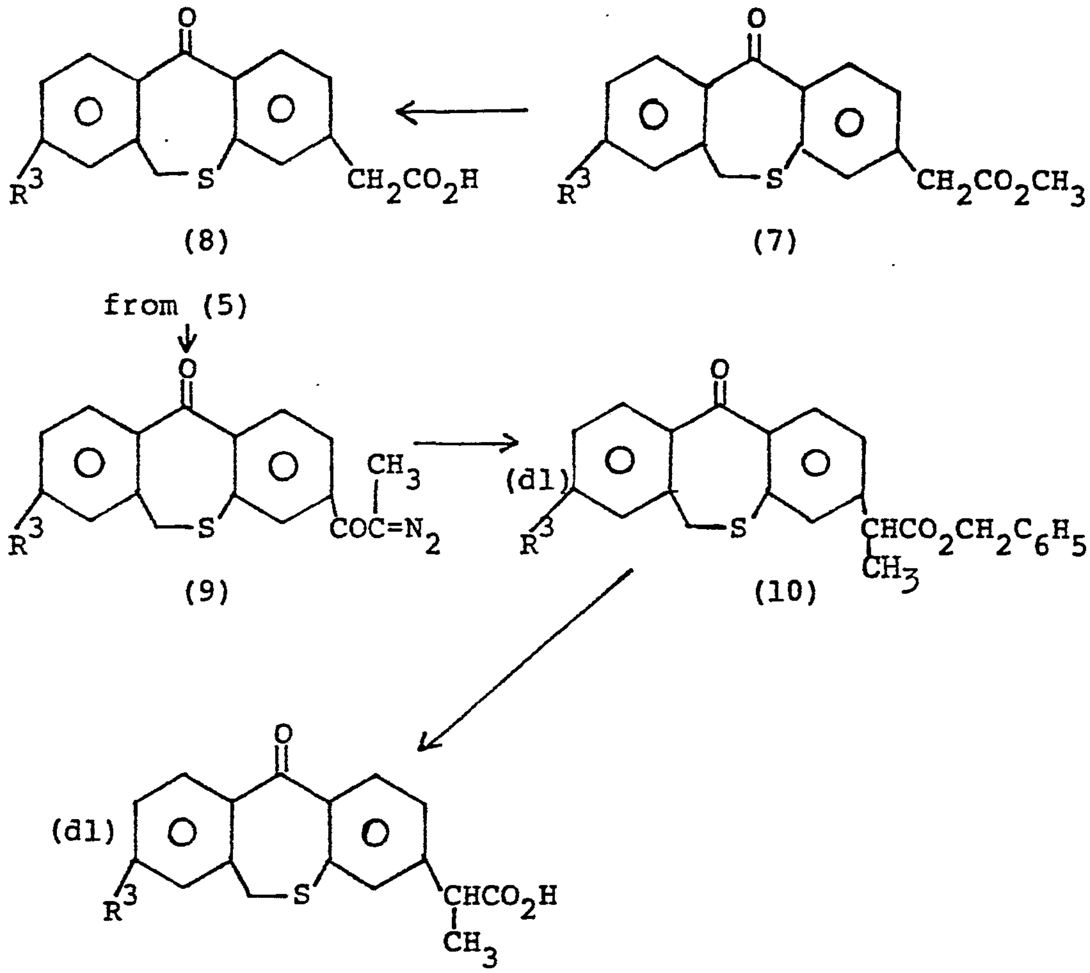


(8) (7) (9) (10) (11)

wherein $R^3$ is defined as above, namely, chloro and methoxy.

Diisopropyl nitroterephthalate (1) is prepared by esterifying nitroterephthalic acid (a) with isopropanol, the isopropanol serving both as reactant and solvent, in the presence of hydrogen chloride at a temperature of from about 25°C. to the reflux temperature of the reaction mixture for from about 48 to about 200 hours. Ordinarily, it is preferred to carry out the reaction at reflux for from about 48 hours to about 96 hours.

Diisopropyl nitroterephthalate (1) is then treated with the $R^3$ substituted benzyl mercaptan and sodium hydride in the presence of a suitable organic solvent,

e.g., dimethylformamide, dimethylsulfoxide, and the like, at temperature of from about -40°C. to about 50°C, preferably , from about -35°C. to about 20°C., for from about one hour to about ten hours preferably from about two hours to about three hours, to obtain diisopropyl ($R^3$ substituted benzylthio)-terephthalate (2).

Base hydrolysis of (2) yields ($R^3$ substituted benzyl thio)-terephthalic acid (3). This reaction is carried out by treating (2) with a base, e.g., potassium hydroxide, sodium hydroxide, and the like, in the presence of water, and an organic solvent, e.g., methanol, ethanol, propanol, and the like, at a temperature of from about 40°C. to the reflux temperature of the reaction mixture, for from about one to about twelve hours. Preferably the hydrolysis reaction is carried out using aqueous methanolic potassium hydroxide at reflux temperature.

($R^3$ substituted benzylthio)-terephthalyl chloride (4) is obtained by treating (3) with thionyl chloride at a temperature of from about 25°C. to the reflux temperature of the reaction mixture for about one to about six hours. It is preferred to carry out this reaction at reflux temperature.

The cyclization reaction by which (4) is converted to 8-$R^3$ substituted-6,11-dihydrodibenzo-[b.e.l-thiepin-11-one-3-carbonyl chloride (5) is carried out by treating (4) with nitromethane and aluminum chloride in the presence of a suitable organic solvent, e.g., methylene chloride, carbon disulfide, o-dichlorobenzene, and the like, at a temperature of from about 15°C. to about 35°C. for from about one-half to about 24 hours, preferably at from about 20°C. to about 25°C., for from about four to about twelve hours. The preferred solvent is methylene chloride.

The conversion of (5) to 3-diazoacetyl-8-$R^3$ substituted-6,11-dihydrodibenzo-[b.e.]-thiepin-11-one (6) is carried out by treating (5) with diazomethane, in an organic solvent, e.g., methylene chloride, ether, carbon tetrachloride, and the like, or mixtures thereof, at a temperature of from about -30°C. to about 10°C., for from about fifteen minutes to about twelve hours. Preferably this reaction is carried out at a temperature of from about -5°C. to about 5°C. for from about fifteen to about sixty minutes.

The rearrangement of (6) to methyl 8-$R^3$ substituted 6,11-dihydrodibenzo-[b.e.]-thiepin-11-one-3-acetate (7) is carried out by intimately mixing (6) with methanol at a temperature of from about 50°C. to the reflux temperature of the mixture, followed by the addition, in portions, of a silver salt, e.g., silver benzoate, dissolved or suspended in a solvent, e.g., methanol or triethylamine. Likewise, other 3-esters, otherwise corresponding to (7) are obtained by substituting the appropriate alkyl alcohol, containing 2 to 12 carbon atoms, for methanol.

The hydrolysis of (7) to 8-$R^3$ substituted-6,11 dihydrodibenzo-[b.e.]-thiepin-11-one-3-acetic acid (8) is carried out by treating (7) with a base, e.g., potassium hydroxide, sodium hydroxide, sodium carbonate, and the like, in the presence of water and an organic solvent, e.g., methanol, ethanol, propanol, and the like, at a temperature of from about 0°C. to about 70°C for from about one to about 24 hours. This hydrolysis reaction is preferably carried out using aqueous methanolic potassium hydroxide at a temperature of from about 20°C. to about 30°C. Likewise the other 3-esters, obtained as described above, are hydrolyzed to the 3-acetic acid (8). The temperature for the hydrolysis will depend on the particular ester to be hydrolyzed and

thus can range from about 0°C. to reflux.

Treatment of 8-$R^3$ substituted-6,11-dihydrodibenzo [b.e.]-thiepin-11-one-3-carbonyl chloride (5) with diazomethane in an organic solvent, e.g., ether, methylene chloride, carbon tetrachloride, and the like, at a temperature of from about -30°C. to about -10°C. is productive of 3-(α-diazopropionyl)-8-$R^3$ substituted-6,11 dihydrodibenzo-[b.e.]-thiepin-11-one (9). It is preferred to carry out this reaction using ethereal diazoethane at a temperature of from about -25°C. to about -20°C.

The rearrangement of (9) to e.g. benzyl (dl)-2-(8-$R^3$ substituted-6,11-dihydrodibenzo-[b.e.]-thiepin-11-one-3-yl) propionate (10) is carried out by treating (9) with a high boiling alcohol, e.g., benzyl alcohol, in the presence of an organic amine, e.g., collidine, quinoline, diethylaniline, and the like, at a temperature of from about 160°C. to about 190°C., for about one minute to about one hour, preferably from about 170°C. to about 175°C. for from about two to about thirty minutes.

The free acid, (dl)-2-($R^3$-substituted 6,11-dihydro dibenzo-[b.e.]-thiepin-11-one-3-yl) propionic acid (11), is obtained by treating the propionate (10) in an organic solvent, e.g., methanol, ethanol, aqueous tetrahydrofuran, and the like, with a base, e.g., an alkali metal hydroxide, at about room temperature to reflux for from about fifteen minutes to about 10 hours, preferably for about 30 minutes to about two hours.

The preparation of the compounds of Formula (I), when R is other than a pharmaceutically acceptable salt,

is illustrated by the following reaction scheme:

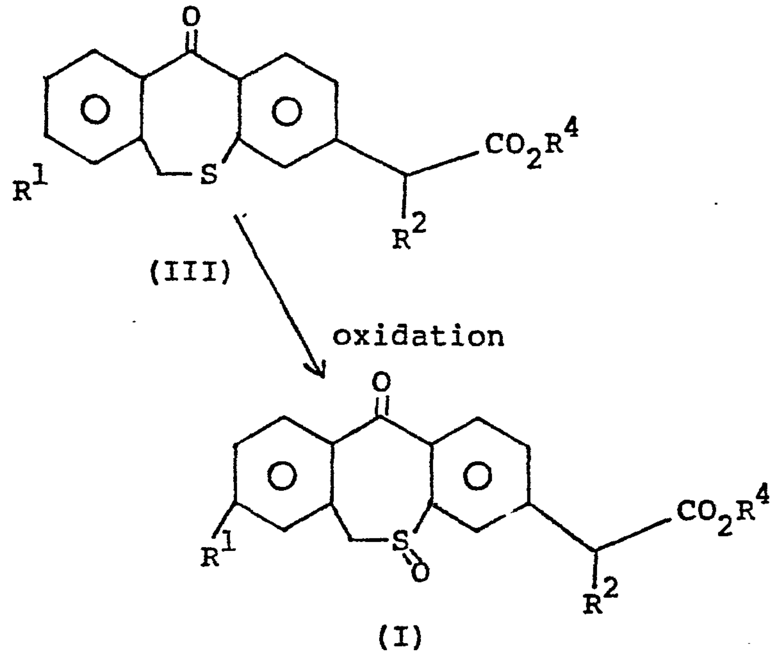


wherein $R^4$ is hydrogen or an "alkyl" group containing from one to twelve carbon atoms as defined above.

The compounds of Formula (III) when $R^1$ is chloro or methoxy, are prepared as described above and more fully below. The compounds of Formula (III) when $R^1$ is hydrogen are prepared as described in my United States Patent Nos. 4,000,288 and 4,000,308, both issued December 28, 1976, which are hereby incorporated by reference and made a part hereof.

The compounds of Formula (I) are obtained by oxidizing the compounds of Formula (III) with an oxidizing agent such as m-chloroperbenzoic acid, perbenzoic acid, peracetic acid, trifluoroperacetic acid, and the like, preferably in the presence of a solvent, e.g., methylene chloride, chloroform, benzene, dimethoxyethane, and the like, or a combination thereof, at a temperature of from about -10°C. to about 50°C., preferably from about 20°C. to about 40°C., for from

about 2 minutes to about 1 hour, preferably from about 5 minutes to about 20 minutes.

Upon their preparation, the free acids of Formula (I) and (II), when R is hydrogen, can be converted to their corresponding esters and acid addition salts.

The salt derivatives of the acid compounds of Formula (I) and (II), are prepared by treating these free acids with an appropriate amount of a pharmaceutically acceptable base. Representative pharmaceutically acceptable bases are sodium hydroxide, potassium hydroxide, lithium hydroxide, ammonium hydroxide, calcium hydroxide, magnesium hydroxide, ferrous hydroxide, zinc hydroxide, copper hydroxide, manganous hydroxide, aluminum hydroxide, ferric hydroxide, manganic hydroxide, isopropylamine, trimethylamine, diethylamine, triethylamine, tripropylamine, ethanolamine, 2-dimethylaminoethanol, 2-diethylaminoethanol, tromethamine, lysine, arginine, histidine, caffeine, procaine, hydrabamine, choline, betaine, ethylenediamine, glucosamine, methylglucamine, theobromine, purines, piperazine, piperidine, N-ethylpiperidine, polyamine resins and the like. The reaction is conducted in water, alone or in combination with an inert, water-miscible organic solvent, at a temperture of from about 0°C. to about 100°C., preferably at room temperature. Typical inert, water-miscible organic solvents include methanol, ethanol, isopropanol, butanol, acetone, dioxane or tetrahydrofuran. The molar ratio of compounds of Formulas (I) and (II), to base are chosen to provide the ratio desired for any particular salt. For preparing, for example, the calcium salts or magnesium salts of the compounds of Formula (I) and (II) the free acid starting material of Formula (I) and (II)

can be treated with at least one-half molar equivalent of pharmaceutically acceptable base to yield a neutral salt. When the aluminum salts of the compounds of Formulas (I) and (II) are prepared, at least one-third molar equivalent of the pharmaceutically acceptable base are employed if a neutral salt product is desired.

In the preferred procedure, the calcium salts and magnesium salts of the compounds of Formulas (I) and (II) can be prepared by treating the corresponding sodium or potassium salts of the compound of Formula (I) and (II) with at least one-half molar equivalent of calcium chloride or magnesium chloride, respectively, in an aqueous solution, alone or in combination with an inert water-miscible organic solvent, at a temperature of from about 20°C. to about 100°C. Preferbly, the aluminum salt of the compounds of Formulas (I) and (II) can be prepared by treating the corresponding free acids of the compounds of Formulas (I) and (II) with at least one-third molar equivalent of an aluminum alkoxide, such as aluminum triethoxide, aluminum tripropoxide and the like, in a hydrocarbon solvent, such as benzene, xylene, cyclohexane, and the like at a temperature of from 20°C. to about 115°C. Similar procedures can be used to prepare salts of inorganic bases which are not sufficiently soluble for easy reaction.

The salt products are isolated by conventional means. For example, the reaction mixtures are evaporated to dryness, and the salts can be further purified by conventional methods.

The salt derivatives of the compounds of Formulas (I) and (II) can be reconverted to their respective free acids by acidifying said salts with an acid, preferably an inorganic acid, e.g. hydrochloric

acid, sulfuric acid, and the like, at temperatures of from about 0°C, preferably at room temperature.

The esters of Formulas (I) and (II) are prepared by esterifying the corresponding free acids of Formulas (I) and (II) with an alcohol reagent corresponding to the desired ester, e.g., an alkanol having up to 12 carbon atoms. This reaction is conducted in the presence of a strong acid, such as boron trifluoride, hydrogen chloride, sulfuric acid, p-toluenesulfonic acid, and the like. If the alcohol reagent used in the esterification is a liquid at the reaction temperature, the alcohol reagent can be the reaction solvent. Optionally, the reaction can be carried out in an inert organic solvent in which the free acids of Formulas (I) and (II) and the alcohol reagent are soluble, such as a hydrocarbon solvent, e.g., hexane, iso-octane, decane, cyclohexane, benzene, toluene, xylene; a halogenated hydrocarbon solvent, e.g., methylene chloride, chloroform, dichloroethane; or an ether solvent, e.g., diethyl ether, dibutyl ether, dioxane, tetrahydrofuran; and the like. In the case wherein the alcohol reagent is a solid, the reaction preferably is conducted in a non-aqueous liquid inert organic solvent. The reaction is conducted at from about 0°C to the reflux temperature of the reaction mixture, preferably using hydrogen chloride at a temperature of from 15°C to about 35°C.

The product is isolated by conventional means such as diluting the reaction mixture with water, extracting the resulting aqueous mixture with a water-immiscible inert organic solvent, such as diethyl ether, benzene, methylene chloride, and the like, combining the extracts, washing the extracts with water to neutrality and then evaporating under reduced pressure.

The preferred acid esters of Formulas (I) and (II)

are those ester derivatives prepared from methyl alcohol, ethyl alcohol, propyl alcohol, isopropyl alcohol, butyl alcohol, 2-butyl alcohol, isoamyl alcohol, pentyl alcohol, 2-pentyl alcohol, isopentyl alcohol, hexyl alcohol, 2-hexyl alcohol, isohexyl alcohol, heptyl alcohol, 2-heptyl alcohol, isoheptyl alcohol, octyl alcohol, 2-octyl alcohol, isooctyl alcohol, nonyl alcohol, 2-nonyl alcohol, isononyl alcohol, decyl alcohol, 2-decyl alcohol, isodecyl alcohol, undecyl alcohol, dodecyl alcohol, and the like.

Alternatively, the esters of Formulas (I) and (II) can be prepared by transesterification, according to methods known in the art. It is preferred in preparing the esters via transesterification to go from a lower ester to a higher ester, e.g., from the methyl ester, for example, to the isoamyl ester, for example. However, by using a substantial excess of a lower alcohol, a higher ester can be transesterified to a lower ester; thus, for example, by using a substantial excess of ethanol, the hexyl ester is converted by the transesterification to the ethyl ester.

The compounds of Formulas (I) and (II) and the esters and pharmaceutically acceptable salts thereof, are useful as anti-inflammatory agents, platelet aggregation inhibitors, fibrinolytic agents, and as smooth muscle relaxants. The compounds of Formulas (I) and (II) and the esters and pharmaceutically acceptable salts thereof, can be used both prophylactically and therapeutically.

The compounds of Formulas (I) and (II) and the esters and pharmaceutically acceptable salts thereof, exhibit anti-inflammatory, analgesic and anti-pyretic activities. Accordingly, the compositions containing these compounds are useful in the treatment and elimination of inflammation such as inflammatory

conditions of the muscular skeletal system, skeletal joints and other tissues, for example, in the treatment of inflammatory conditions such as rheumatism, concussion, laceration, arthritis, bone fractures, post-traumatic conditions, and gout. In those cases in which the above conditions include pain and pyrexia coupled with inflammation, the instant compounds are useful for the relief of these conditions as well as the inflammation.

The S-oxide of Formula (I) when administered to primates exhibit, for example, sustained activity, as compared to the corresponding non-S-oxides. Without wishing to be bound thereby, applicant theorizes that in primates the S-oxides must first biochemically be converted into the corresponding non-S-oxides, and because of the necessity of this biochemical conversion step, the activity of the S-oxides is sustained.

Administration of the active compound of Formulas (I) and (II) and the esters and pharmaceutically acceptable salts thereof, in an appropriate pharmaceutical composition can be via any of the accepted modes of administration of agents for the treatment of inflammation, pain, or pyrexia, or the prophylaxis thereof. Thus, administration can be for example, orally, parenterally, or topically, in the form of solid, semi-solid or liquid dosage forms, such as, for example, tablets, suppositories, pills, capsules, powders, liquids, suspensions, creams, lotions, ointments, or the like, preferably in unit dosage forms suitable for simple administration of precise dosages. The compositions will include a conventional pharmaceutical carrier or excipient and an active compound of Formulas (I) and (II) and the esters and pharmaceutically acceptable salts thereof, and, in

addition, may include other medicinal agents, pharmaceutical agents, carriers, adjuvants, etc.

The preferred manner of administration, for the conditions detailed above, is oral using a convenient daily dosage regimen which can be adjusted according to the degree of affliction. Generally, a daily dose of from about 0.05 mg. to about 10 mg. of the active compound of Formulas (I) and (II) and the esters and pharmaceutically acceptable salts thereof, per kilogram of body weight is used. Most conditions respond to treatment comprising a dosage level of the order of from about 0.25 mg. to about 3 mg. per kilogram of body weight per day. For such oral administration, a pharmaceutically acceptable non-toxic composition is formed by the incorporation of any of the normally employed excipients, such as, for example, pharmaceutical grades of mannitol, lactose, starch, magnesium stearate, sodium saccharin, talcum, cellulose, glucose, sucrose, magnesium carbonate, and the like. Such compositions take the form of solutions, suspensions, tablets, pills, capsules, powders, sustained release formulations and the like.

The active compound of Formulas (I) and (II) and the esters and pharmaceutically acceptable salts thereof, may be formulated into a suppository using, for example, polyalkylene glycols, for example, propylene glycol, as the carrier. Liquid pharmaceutically administerable compositions can, for example, be prepared by dissolving, dispersing, etc. an active compound, as described above, and optional pharmaceutical adjuvants in a carrier, such as, for example, water, saline, aqueous dextrose, glycerol, ethanol, and the like, to thereby form a solution or suspension. If desired, the pharmaceutical composition to be administered may also contain minor amounts of non-toxic

auxiliary substances such as wetting or emulsifying agents, pH buffering agents and the like, such as for example, sodium acetate, sorbitan monolaurate, triethanolamine oleate, etc.

Actual methods of preparing such dosage forms are known, or will be apparent, to those skilled in this art; for example, see Remington's Pharmaceutical Sciences, Mack Publishing Company, Easton, Pennsylvania, 14th. Edition, 1970. The composition to be administered will, in any event, contain a quantity of the active compound(s) in a pharmaceutically effective amount for relief of the particular condition being treated in accordance with the teachings of this invention.

The compounds of Formulas (I) and (II) and the esters and pharmaceutically acceptable salts thereof, described above, are also uterine smooth muscle relaxants and thus are useful as agents for maintaining the pregnancy of pregnant mammals, for the benefit of the mother and/or the fetus, until termination of the pregnancy is considered, from a medical point of view, to be favorble, or more favorable, for the mother and/or the fetus. It should be understood, however, that in certain instances, for example where parturition has already begun (i.e., the mother is experiencing uterine contractions, especially near full term), that administration of the compounds herein described may not maintain the pregnant state for an indefinite period of time. Rather, in such instances, the pregnancy will, most probably, be slightly "prolonged", a factor which may be advantageous to either the mother and/or the fetus.

In particular, the compounds of Formulas (I) and (II) and the esters and pharmaceutically acceptable salts thereof, are used as agents for delaying the onset of, or for postponing, parturition. As used in this

application, the phrase "to delay the onset of parturition" is intended to cover that delay in parturition caused by the administration of the compounds of Formulas (I) and (II) and the esters and pharmaceutically acceptable salts thereof, at any time before uterine muscle contractions have begun. Thus, it is intended that the aforementioned phrase cover abortion prevention early in pregnancy (i.e., before the fetus is "viable") as well as delaying premature parturition, a term which sometimes is used with reference to that premature labor experienced later in the pregnancy when the fetus is considered to be "viable". In either case, the agents are administered as prophylactic agents in that such administration tends to prevent the onset of parturition. This administration is particularly useful in the treatment of women having a history of spontaneous abortion, miscarriage or premature delivery (i.e., delivery prior to full term). Such administration is also useful where there are clinical indications that the pregnancy might be terminated prior to that time and is considered favorable to the mother and/or the fetus.

With respect to animals, this treatmeant can also be utilized to synchronize the deliveries from a group of pregnant animals to happen at or about the same time, or to happen at or about a desired time and/or place, when the births can be handled with greater facility.

As used in this application, the phrase "postponing parturition" is intended to cover that delay in parturition caused by the administration of the compounds of Formulas (I) and (II) and the esters and pharmaceutically acceptable salts thereof after uterine muscle contractions have begun. The condition of the patient, including the time within the gestation period when the

contractions have begun, the severity of the contractions and how long the contractions have taken place will affect the results achieved with the administration of the compounds of Formulas (I) and (II) and the esters and pharmaceutically acceptable salts thereof. For example, the effect can be to reduce the intensity and/or the duration of the contractions (the actual act of parturition being "prolonged"), or to stop the contractions altogether. In either case, the effect will be to prolong the gestation period although, depending upon the condition of the patient as described above, the effect may either be slight or, under appropriate circumstances, somewhat greater. Such administration may be to prevent spontaneous abortion, to cause the delivery to be more easily accomplished and/or less painful to the mother, or to occur at a more appropriate time and/or place.

In all cases, administration of the compounds of Formulas (I) and (II) and the esters and pharmaceutically acceptable salts thereof, for the purposes set forth herein, should be consistent with best and/or accepted medical (or veterinary) practices so as to maximize the benefits to the mother and the fetus. For example, administration should not be continued so long past full term that the fetus dies in utero.

In the practice of the methods of the present invention, a therapeutically effective amount of a compound of Formulas (I) and (II) and the esters and pharmaceutically acceptable salts thereof, or a pharmaceutical composition containing a compound of Formulas (I) and (II) and the esters and pharmaceutically acceptable salts thereof, is administered to the pregnant mammal via any of the usual and acceptable methods known in the art. The compound can be administered either singly or in combination with

another compound or compounds, as defined above, or other pharmaceutical agents, carriers, adjuvants, etc. Such compound(s) or compositions can be administered orally, parenterally, either in the form of solid, semi-solid, or liquid dosage forms. Typically, administration is by a pharmaceutical composition containing the pharmaceutically active compound and one of more pharmaceutical carriers or adjuvants.

The administerable pharmaceutical composition may take the form of oral tablets, vaginal or uterine tablets or suppositories, pills, capsules, lioquid solutions, suspensions, or the like, preferably in unit dosage forms suitable for simple administration of precise dosages. Conventional non-toxic solid carriers include, for example, pharmaceutical grades of mannitol, lactose, starch, magnesium stearate, sodium saccharin, talcum, cellulose, glucose, sucrose, magnesium carbonate, and the like. The active compound as defined above may be formulated as suppositories using, for example, polyalkylene glycols, for example, propylene glycol, as the carrier. Liquid pharmaceutically administerable compositions can, for example, be prepared by dissolving, dispersing, etc. an active compound as defined above and optional pharmaceutical adjuvants in a carrier, such as, for example, water, saline, aqueous dextrose, glycerol, ethanol, and the like, to thereby form a solution or suspension. If desired, the pharmaceutical composition to be administered may also contain minor amounts of non-toxic auxiliary substances such as wetting or emulsifying agents, pH buffering agents and the like, for example, sodium acetate, sorbitan monolaurage, triethanolamine oleate, etc. Actual methods of preparing such dosage forms are known, or will be apparent, to those skilled

in this art; for example, see Remington's Pharmaceutical Sciences, Mack Publishing Company, Easton, Pennsylvania, 14th. Edition, 1970. The composition or formulation to be administered will, in any event, contain a quantity of the active compound(s) in an amount effective to delay the onset of parturition or to postpone parturition if uterine contractions have already begun. Generally a daily dose of from about 5 mg. to about 250 mg. of the active compound per kilogram of body weight will be administered, with administration being a single daily dose or up to three or four smaller doses regularly given throughout the day. The amount of active compound administered will, of course, depend on its relative activity.

## Description of Specific Embodiments

The following specific description, recited in the Preparations and Examples below, is given to enable those skilled in this art to more clearly understand and practice the present invention. It should not be considered as a limitation upon the scope of the invention but merely as being illustrative and representative thereof.

It is to be understood that isolation of the compounds described herein can be effected by any suitable separation or purification procedure, such as, for example, extraction, filtration, evaporation, distillation, crystallization, thin-layer chromatography or column chromatography, or a combination of these procedures. Illustrations of suitable separation and isolation procedures can be had by reference to the Preparations and Examples described herein below. However, other equivalent separation or isolation procedures, could, of course, also be used.

Where necessary, examples (and preparations) are repeated to prepare additional material for later

examples (and preparations); and unless otherwise specified, the reactions are carried out at room temperature (20°C. to 30°C.).

Preparation 1

200 G. of nitroterephthalic acid (a) is dissolved in 1 liter of isopropanol and the thus-obtained solution is saturated with hydrogen chloride and refluxed for three days. (During this period, hydrogen chloride is passed into the solution occasionally in order to maintain the concentration thereof.) The reaction solution is then cooled and the isopropanol is removed by evaporation under reduced pressure to give a residue which is dissolved in 500 ml. of methylene chloride. The resultant solution is washed with 10% acqueous sodium carbonate, and the organic layer obtained is dried over magnesium sulfate, followed by removal of the solvent in vacuo to give 245 g. (yield 87.5%) of diisopropyl nitroterephthalate (1), an oil; IR: $\nu_{max.}^{CHCl_3}$ 1724, 1542, 1350 cm.$^{-1}$; NMR: $\delta_{TMS}^{CDCl_3}$ 1.35 (6H, d), 1.40 (6H, d), 5.24 (1H, 7 lines), 5.27 (1H, 7 lines), 7.71 (1H, d), 8.24 (1H, dd), 8.43 ppm (1H, d).

Preparation 2

5.1 G. of sodium hydride is slowly added to a cooled (-20°C.) solution of 23.5 m. of m-chlorobenzyl mercaptan in 100 ml. of dimethylformamide. The resultant solution is cooled to -30°C. and there is added thereto 53 g. of diisopropyl nitroterephthalate (1) in 100 ml. of dimethylformamide. After one hour at -30°C. and two hours at 0°C., the reaction mixture is poured into water, the precipitate is filtered off, washed with water and dried to yield 66% of crude diisopropyl-(m-chlorobenzylthio)-terephthalate (2), a sample of which, following recrystallization from pentane, melted at 63-64°C.

In like manner substituting a stoichiometric

equivlent account of m-methoxybenzyl mercaptan for m-chlorobenzyl mercaptan there is obtained crude diisopropyl-(m-methoxybenzylthio)-terephtalate, in 78% yield, having a melting point of 52-53°C.

Preparation 3

The crude diisopropyl-(m-chlorobenzylthio)-terephthalate (2) obtained in Preparation 2 is refluxed with 500 ml. methanol, 25 g. of potassium hydroxide and 50 ml. of water for two hours. The reaction mixture is then concentrated to a small volume, cooled, diluted with water and filtered through diatomaceous earth (Celite). The thus-obtained filtrate is acidified with 4N hydrochloric acid and the precipitate which formed is collected by filtration and dried in an oven at 90-100°C. to yield (m-chlorobenzylthio)-terephthalic acid (3), in 98% yield, having a melting point of 279°-280°C.

In like manner substituting stoichiometric equivalent amount of diisopropyl-(m-methoxybenzylthio)-terephthalate for diisopropyl-(m-chlorobenzylthio)-terephthalate there is obtained (m-methoxybenzylthio)-terephthalic acid, in 98% yield, having a melting point of 255-256°C.

Preparation 4

10 G. of (m-chlorobenzylthio)-terephthalic acid (3) is treated with 10 ml. of thionyl chloride and the reaction mixture is refluxed for four hours. After removal of the excess thionyl chloride in vacuo, the residue obtained is slurried with hexane and the solid product is filtered off to yield (m-chlorobenzylthio)-terephthalyl chloride (4), an oil, in 98% yield, having an IR: $\nu_{max.}^{film}$ 1750 $cm^{-1}$.

In like manner substituting a stoichiometric equivalent amount of (m-methoxybenzylthio)-terephthalic acid for (m-chlorobenzylthio)-terephthalic acid there is

obtained (m-methoxybenzylthio)-terephthalyl chloride, in 94% yield, having a melting point of 88-90°C.

Preparation 5

10.2 G. of (m-chlorobenzylthio)-terephthalyl chloride (4) in 100 ml. of methylene chloride is added to a solution of 14.75 g. of aluminum chloride in 100 ml. of methylene chloride containing 10.51 ml. of nitromethane. After five hours at 25°C., 16.5 ml. of saturated aqueous sodium chloride is added with vigorous stirring. The inorganic salts which precipitate are filtered off and the filtrate is evaporated to dryness to give a solid residue which is slurried with ether. The ether slurry is filtered to yield 8-chloro-6,11,dihydrodibenzo-[b.e.]-thiepin-11-one-3-carbonyl chloride (5), in 50% yield, having a melting point of 102-103°C.

In like manner substituting a stoichiometric equivalent amount of (m-methoxybenzylthio)-terephthalyl chloride for (m-chlorobenzylthio)-terephthalyl chloride there is obtained 8-methoxy-6,11-dihydrodibenzo-[b.e.]-thiepin-11-one-3-carbonyl chloride, in 52% yield, having a melting point of 117-118°C.

Preparation 6

A solution of 11 g. of 8-chloro-6,11-dihydrodibenzo-[b.e.]-thiepin-11-one-3-carbonyl chloride (5) in 100 ml. of methylene chloride is slowly added to excess diazomethane (prepared from 20 g. N-nitroso N-methylurea) in 200 ml. of ether solution. After two hours the reaction mixture is concentrated to about 50 ml. by boiling off the solvent, followed by cooling. The cooled reaction mixture is filtered to yield 3-diazoacetyl-8-chloro 6,11-dihydrodibenzo-[b.e.]thiepin-11-one (6), in 75% yield, having a melting point of 155°C., with decomposition.

In like manner substituting 8-methoxy-6,11-dihydrodibenzo-[b.e.]-thiepin-11-one-3-carbonyl chloride

for 8-chloro-6,11-dihydrodibenzo-[b.e.]-thiepin-11-one-3-carbonyl chloride there is obtained 3-diazoacetyl-8-methoxy-6,11-dihydrodibenzo-[b.e.]-thiepin-11-one, in 73% yield, having a melting point of 160°C., with decomposition.

EXAMPLE 1

9.5 G. of 3-diazoacetyl-8-chloro-6,11-dihydro-dibenzo-[b.e.]-thiepin-11-one (6) is suspended in 500 ml. of methaonl and the suspension is stirred vigorously and refluxed. 2 G. of silver benzoate suspended in 20 ml. of methanol is added in 2 ml. portions over 50 minutes. The reaction mixture is refluxed for 15 hours, cooled and the solvent removed at the pump and following chromatography of the residue on 400 g. of silica gel there is obtained methyl 8-chloro-6,11-di-hydrodibenzo-[b.e.]-thiepin-11-one-3-acetate (7), in 48% yield, having a melting point (ether) of 101-102°C.

In like manner substituting a stoichiometric equivalent amount of 3-diazoacetyl-8-methoxy-6,11-dihydrodibenzo-[b.e.]thiepin-11-one for 3-diazo-acetyl-8-chloro-6,11-dihydrodibenzo-[b.e.]-thiepin-11-one there is obtained methyl 8-methoxy-6,11-dihydrodi-benzo-[b.e.]-thiepin-11-one-3-acetate, in 38% yield, having a melting point of 97-98°C.

Similarly, by substituting other alkyl alcohols, containing 2 to 12 carbon atoms, for methanol, in the procedure of this Example there are obtained, for example,

ethyl 8-chloro- and 8-methoxy-6,11-dihydro-dibenzo-[b.e.]-thiepin-11-one-3-acetate,

propyl 8-chloro- and 8-methoxy-6,11-dihydrodibenzo-[b.e.]-thiepin-11-one-3-acetate,

isopropyl 8-chloro- and 8-methoxy-6,11-dihydrodi-benzo-[b.e.]-thiepin-11-one-3-acetate,

hexyl 8-chloro- and 8-methoxy-6,11-dihydrodibenzo-[b.e.]-thiepin-11-one-3-acetate,

nonyl 8-chloro- and 8-methoxy-6,11-dihydrodibenzo-[b.e.]-thiepin-11-one-3-acetate,

dodecyl 8-chloro- and 8-methoxy-6,11-dihydrodibenzo-[b.e.]-thiepin-11-one-3-acetate, and the like.

EXAMPLE 2

7.0 G. of methyl 8-chloro-6,11-dihydrodibenzo-[b.e.]-thiepin-11-one-3-acetate (7) is stirred with 200 ml. of methanol and a solution of 2.0 g. of potassium hydroxide in 5 ml. of water is added. After one hour, the solution is clarified by filtration and the filtrate is acidified with 3N aqueous hydrochloric acid and diluted with 400 ml. of water. The precipitated solid is filtered off, dried in an oven at about 80°C. and recrystallized from benzene:hexane to yield 8-chloro-6,11-dihydrodibenzo-[b.e.]-thiepin-11-one-3-acetic acid (8), in 90% yield, having a melting point of 190-191°C.

In like manner substituting a stoichiometric equivalent amount of methyl 8-methoxy-6,11-dihydrodibenzo-[b.e.]-thiepin-11-one-3-acetate for methyl 8-chloro-6,11-dihydrodibenzo-[b.e.]-thiepin-11-one-3-acetate there is obtained 8-methoxy-6,11-dihydrodibenzo-[b.e.]-thiepin-11-one-3-acetic acid, in 88% yield, having a melting point of 168-169°C.

Similarly, 8-chloro- and 8-methoxy-6,11-dihydrodibenzo-[b.e.]-thiepin-11-one-3-acetic acids are obtained by substituting, in place of methyl 8-chloro-and 8-methoxy-6,11-dihydrodibenzo-[b.e.]-thiepin-11-one-3-acetates, the other 3-esters obtained in Example 1, for example,

ethyl 8-chloro- and 8-methoxy 6,11-dihydrodibenzo-[b.e.]-thiepin-11-one-3-acetate,

propyl 8-chloro- and 8-methoxy-6,11-dihydrodibenzo-[b.e.]-thiepin-11-one-3-acetate,

isopropyl 8-chloro- and 8-methoxy-6,11-dihydrodibenzo-[b.e.]-thiepin-11-one-3-acetate,

hexyl 8-chloro- and 8-methoxy-6,11-dihydrodibenzo-[b.e.]-thiepin-11-one-3-acetate,

nonyl 8-chloro- and 8-methoxy-6,11-dihydrodibenzo-[b.e.]-thiepin-11-one-3-acetate,

dodecyl 8-chloro- and 8-methoxy-6,11-dihydrodibenzo-[b.e.]-thiepin-11-one-3-acetate, and the like.

EXAMPLE 3

A solution of 2.5 g. of 8-chloro-6,11-dihydrodibenzo-[b.e.]-thiepin-11-one-3-carbonyl chloride (5) in 30 ml. of methylene chloride is added to a solution of diazoethane (prepared from 20 g. N-ethyl N-nitrosourea) in 150 ml. of ether at -20°C. The reaction mixture is allowed to warm to room temperature and most of the solvent is blown off in a stream of nitrogen, followed by filtration to yield 3-(α-diazopropionyl)-8-chloro-6,11-dihydrodibenzo-[b.e.]-thiepin-11-one (9), in 58% yield, having a melting point of 100°C., with decomposition.

In like manner substituting a stoichiometric equivalent amount of 8-methoxy-6,11-dihydrodibenzo-[b.e.]-thiepin-11-one-3-carbonyl chloride for 8-chloro-6,11-dihydrodibenzo-[b.e.]-thiepin-11-one-3-carbonyl chloride there is obtained 3-(α-diazopropionyl)-8-methoxy-6,11-dihydrodibenzo-[b.e.]-thiepin-11-one, in 40% yield, having a melting point of 101°C. with decomposition.

EXAMPLE 4

1.1 G. of 3-(α-diazopropionyl)-8-chloro-6,11-dihydrodibenzo-[b.e.]-thiepin-11-one (9) suspended in

3 ml. of collidine is added to a mixture of five ml. of benzyl alcohol and five ml. of collidine maintained at 170-175°C. After five minutes, the reaction mixture is cooled and the solvents are distilled off in vacuo, followed by chromatography of the residue on silica gel to yield an oil consisting mainly of benzyl (dl)-2-(8-chloro-6,11-dihydrodibenzo-[b.e.]-thiepin-11-one-3-yl) propionate (10).

In like manner substituting a stoichiometric equivalent amount of 3-(α-diazopropionyl)-8-methoxy-6,11-dihydrodibenzo-[b.e.]-thiepin-11-one for 3-(α-diazopropionyl)-8-chloro-6,11-dihydrodibenzo-[b.e.]-thiepin-11-one, there is obtained an oil consisting mainly of benzyl (dl)-2-(8-methoxy-6,11-dihydrodibenzo-[b.e.]-thiepin-11-one-3-yl) propionate.

EXAMPLE 5

The oil consisting mainly of benzyl (dl)-2-(6,11-dihydrodibenzo-[b.e.]-thiepin-11-one-3-yl) propionate (10), obtained in Example 4, is taken up in 25 ml. of methanol and treated with 0.3 g. of potassium hydroxide in 2 ml. of water. The reaction mixture is refluxed for one hour, cooled and poured into 200 ml. of water. The thus-obtained mixture is acidified with 10 ml. of dilute hydrochloric acid and extracted with 50 ml. of ethyl acetate. The organic layer is separated and washed with 25 ml. of 10% aqueous sodium carbonate solution. The basic aqueous layer is acidified with 10 ml. of dilute 3N hydrochloric acid and extracted with 50 ml. of ether. The ether layer is washed, dried, and evaporated to yield a residue which is recrystallized from methanol/water to yield (dl)-2-(8-chloro-6,11-dihydrodibenzo-[b.e.]-thiepin-11-one-3-yl) propionic acid (11), in 50% yield, having a melting point of 152-153°C.

In like manner substituting the oil consisting mainly of benzyl (dl)-2-(8-methoxy-6,11-dihydrodibenzo-[b.e.]-thiepin-11-one-3-yl)propionate, obtained as described in Example 4, for its corresponding 8-chloro compound, there is obtained (dl)-2-(8-methoxy-6,11-dihydrodibenzo-[b.e.]-thiepin-11-one-3-yl) propionic acid, in 66% yield, having a melting point of 132-133°C.

EXAMPLE 6

250 Mg. of 8-chloro 6,11-dihydrodibenzo-[b.e.]-thiepin-11-one-3-acetic acid (8) is dissolved in 30 ml. of isoamyl alcohol and the solution is saturated with hyrogen chloride. After 90 minutes, the excess alcohol is distilled off in vacuo and the residue is purified by preparative thin-layer chromatography to yield 8-chloro 6,11-dihydrodibenzo-[b.e.]-thiepin-11-one-3-acetate.

In like manner substituting a stoichiometric equivalent amount of 8-methoxy-6,11-dihydrodibenzo-[b.e.]-thiepin-11-one-3-acetic acid for 8-chloro-6,11-dihydrodibenzo-[b.e.]-thiepin-11-one-3-acetic acid there is obtained 8-methoxy-6,11-dihydrodibenzo-[b.e.]-thiepin-11-one-3-acetate.

Likewise other esters, e.g., methyl, ethyl, propyl, isopropyl, hexyl, nonyl, dodecyl, and the like, of 8-chloro- and 8-methoxy-6,11-dihydrodibenzo-[b.e.]-thiepin-11-one-3-acetic acid (8) are obtained by substituting other alcohols, e.g., methyl, ethyl, propyl, isopropyl, hexyl, nonyl, dodecyl alcohol and the like, for isoamyl alcohol.

EXAMPLE 7

150 Mg. of (dl)-2-(8-chloro-6,11-dihydrodibenzo-[b.e.]-thiepin-11-one-3-yl) propionic acid (11) is dissolved in five ml. of methanol and the solution is saturated with hydrogen chloride. After 24 hours, the

excess alcohol is distilled off in vacuo and the residue is purified by chromatography on silica gel to yield methyl (dl)-2-(8-chloro-6,11-dihydrodibenzo-[b.e.]-thiepin-11-one-3-yl) propionate.

In like manner substituting a stoichiometric equivalent amount of (dl)-2-(8-methoxy-6,11-dihydro-dibenzo-[b.e.]-thiepin-11-one-3-yl) propionic acid for (dl)-2-(8-chloro-6,11-dihydrodibenzo-[b.e.]-thiepin-11-one-3-yl)-propionic acid is productive of the methyl ester of (dl)-2-(8-methoxy-6,11-dihydrodibenzo-[b.e.]-thiepin-11-one-3-yl) propionic acid.

EXAMPLE 8

300 Mg. of (dl)-2-(8-chloro-6,11-dihydrodibenzo-[b.e.]-thiepin-11-one-3-yl) propionic acid (11) is dissolved in five ml. of isoamyl alcohol and the solution is saturated with hydrogen chloride. After 24 hours, the excess alcohol is distilled off in vacuo and the residue is purified by chromatography on alumina to yield isoamyl (dl)-2-(8-chloro-6,11-dihydrodibenzo-[b.e.]-thiepin-11-one-3-yl) propionate.

In like manner substituting a stoichiometric equivalent amount of (dl)-2-(-methoxy-6,11-dihydro-dibenzo-[b.e.]-thiepin-11-one-3-yl) propionic acid for (dl)-2-(8-chloro-6,11-dihydrodibenzo-[b.e.]-thiepin-11-one-3-yl) propionic acid there is obtained isoamyl (dl)-2-(8-methoxy-6,11-dihydrodibenzo-[b.e.]-thiepin-11-one-3-yl) propionate.

Likewise other esters, e.g., ethyl, propyl, isopropyl, hexyl, nonyl, dodecyl, and the like, of (dl)-2-(8-chloro-and 8-methoxy-6,11-dihydrodibenzo-[b.e.]-thiepin-11-one-3-yl) propionic acid are obtained by substituting other alcohols, e.g., ethyl, propyl, isopropyl, hexyl, nonyl, dodecyl alcohol, and the like, for isoamyl alcohol.

EXAMPLE 9

A solution of 150 mg. of 6,11-dihydrodibenzo-[b.e.]-thiepin-11-one-3-acetic acid in 1 ml. of in dimethoxyethane is treated with a solution of m-chloroperbenzoic acid (110 mg., 85%) in one ml. of methylene chloride. The reaction mixture is allowed to stand overnight at room temperature and the material which crystallizes is collected by filtration, and recrystallized from ethanol to yield 148 mg. of 6,11-dihydrodibenzo-[b.e]-thiepin-11-one-3-acetic S-oxide, in 93% yield, having a melting point of 185°C.

In like manner substituting a stoichiometric equivalent ammount of

8-chloro-6,11-dihydrodibenzo-[b.e.]-thiepin-11-one-3-acetic acid,

8-methoxy-6,11-dihydrodibenzo-[b.e.]-thiepin-11-one-3-acetic acid,

(dl)-2-(6,11-dihydrodibenzo-[b.e.]-thiepin-11-one-3-yl) propionic acid,

(dl)-2-(8-chloro-6,11-dihydrodibenzo-[b.e.]-thiepin-11-one-3-yl) propionic acid, and

(dl)--2-(8-methoxy-6,11-dihydrodibenzo-[b.e.]-thiepin-11-one-3-yl)propionic acid, for

6,11-dihydrobenzo-[b.e.]-thiepin-11-one-3-acetic acid is productive of

8-chloro-6,11-dihydrodibenzo-[b.e.]-thiepin-11-one-3-acetic acid S-oxide,

8-methoxy-6,11-dihydrodibenzo-[b.e.]-thiepin-11-one-3-acetic acid S-oxide,

(dl)-2-(6,11-dihydrodibenzo-[b.e.]-thiepin-11-one-3-yl) propionic acid S-oxide,

(dl)-2-(8-chloro-6,11-dihydrodibenzo-[b.e.]-thiepin-11-one-3-yl) propionic acid S-oxide, and

(dl)-2-(8-methoxy-6,11-dihydrodibenzo-[b.e.]-thiepin-11-one-3-yl) propionic acid S-oxide, respectively.

Similarly the esters of

6,11-dihydrobenzo-[b.e.]-thiepin-11-one-3-acetic acid,

8-chloro-6,11-dihydrobenzo-[b.e.]-thiepin-11-one-3-acetic acid,

8-methoxy-6,11-dihydrobenzo-[b.e.]-thiepin-11-one-3-acetic acid,

(dl)-2-(6,11-dihydrodibenzo-[b.e.]-thiepin-11-one-3-yl) propionic acid,

(dl)-2-(8-chloro-6,11-dihydrodibenzo-[b.e.]-thiepin-11-one-3-yl) propionic acid, and

(dl)-2-(8-methoxy-6,11-dihydrodibenzo-[b.e.]-thiepin-11-one-3-yl) propionic acid, are converted into their corresponding esters of

6,11-dihydrodibenzo-[b.e.]-thiepin-11-one-3-acetic acid S-oxide,

8-chloro-6,11-dihydrodibenzo-[b.e.]-thiepin-11-one-3-acetic acid S-oxide,

8-methoxy-6,11-dihydrodibenzo-[b.e.]-thiepin-11-one-3-acetic acid S-oxide,

(dl)-2-(6,11-dihydrodibenzo-[b.e.]-thiepin-11-one-3-yl) propionic acid S-oxide,

(dl)-2-(8-chloro-6,11-dihydrodibenzo-[b.e.]-thiepin-11-one-3-yl) propionic acid S-oxide, and

(dl)-2-(8-methoxy-6,11-dihydrodibenzo-[b.e.]-thiepin-11-one-3-yl)propionic acid S-oxide

EXAMPLE 10

300 Mg. of 6,11-dihydrodibenzo-[b.e.]-thiepin-11-one-3-acetic acid S-oxide in five ml. of methanol is titrated with 1N methanolic potassium hydroxide to a faint orange color. The color is discharged by the addition of three mg. of solid 6,11-dihydrodibenzo-[b.e.]-thiepin-11-one-3-acetic acid S-oxide. The solvent is evaporated in vacuo and the residue taken up

in 2 ml. of methanol, followed by precipitation with ether to yield potassium 6,11-dihydrodibenzo-[b.e.]-thiepin-11-one-3-acetic S-oxide.

Likewise other salts, e.g., ammonium and sodium, of 6,11-dihydrodibenzo-[b.e.]-thiepin-11-one-3-acetic acid S-oxide are prepared by substituting ammonium hydroxide and sodium hydroxide for potassium hydroxide.

In like manner the potassium, ammonium and sodium salts of

8-chloro-6,11-dihydrodibenzo-[b.e.]-thiepin-11-one-3-acetic acid S-oxide,

8-methoxy-6,11-dihydrodibenzo-[b.e.]-thiepin-11-one-3-acetic acid S-oxide,

8-chloro-6,11-dihydrodibenzo-[b.e.]-thiepin-11-one-3-acetic acid, and

8-methoxy-6,11-dihydrodibenzo-[b.e.]-thiepin-11-one-3-acetic acid are prepared.

EXAMPLE 11

150 Mg. of (dl)-2-(6,11-dihydrodibenzo-[b.e.]-thiepin-11-one-3-yl) propionic acid S-oxide in one ml. of methanol is titrated with 1N methanolic potassium hydroxide to a faint orange color. A small amount of solid (dl)-2-(6,11-dihydrodibenzo-[b.e.]-thiepin-11-one-3-yl) propionic acid S-oxide is added to decolorize the solution. The solvent is stripped and 5 ml. of toluene is added. The thus obtained toluene solution is evaporated to dryness to give a solid, which is dried at 100°C. in vacuo to yield potassium (dl)-2-(6,11-dihydrodibenzo-[b.e.]-thiepin-11-one-3-yl) propionate S-oxide.

Likewise other salts, e.g., ammonium and sodium, of (dl)-2-(6-11-dihydrodibenzo-[b.e.]-thiepin-11-one-3-yl) propionic acid S-oxide are prepared by substituting, e.g., ammonium hydroxide and sodium hydroxide for

potassium hydroxide.

In like manner the potassium, ammonium and sodium salts of

(dl)-2-(8-chloro-6,11-dihydrodibenzo-[b.e.]-thiepin-11-one3-yl) propionic acid S-oxide,

(dl)-2-(8-methoxy)-6,11-dihydrodibenzo-[b.e.]-thiepin-11-one3-yl) propionic acid S-oxide

(dl)-2-(8-chloro-6,11-dihydrodibenzo-[b.e.]-thiepin-11-one-3-yl) propionic acid, and

(dl)-2-(8-methoxy-6,11-dihydrodibenzo-[b.e.]-thiepin-11-one-3-yl) propionic acid, are prepared.

EXAMPLE 12

300 Mg. of 6,11-dihydrodibenzo-[b.e.]-thiepin-11-one-3-acetic acid S-oxide is dissolved in an excess of 1N aqueous sodium hydroxide and the resultant solution is buffered with 0.3 g. of ammonium chloride. The buffered solution is added to a solution of 200 mg. calcium carbonate in 1N aqueous hydrochloride acid. The precipitate which formed is collected by filtration, washed consecutively with water, dimethoxyethane and ether, to yield calcium 6,11-dihydrodibenzo-[b.e.]-thiepin-11-one-3-acetate S-oxide.

Likewise magnesium 6,11-dihydrodibenzo-[b.e.]-thiepin-11-one-3-acetate S-oxide is prepared by substituting magnesium carbonate for calcium carbonate.

In like manner the calcium and magnesium salts of

8-chloro-6,11-dihydrodibenzo-[b.e.]-thiepin-11-one-3-acetic S-oxide,

8-methoxy-6,11-dihydrodibenzo-[b.e.]-thiepin-11-one-3-acetic S-oxide,

8-chloro-6,11-dihydrodibenzo-[b.e.]-thiepin-11-one-3-acetic acid, and

8-methoxy-6,11-dihydrodibenzo-[b.e.]-thiepin-11-one-3-acetic acid, are prepared.

EXAMPLE 13

175 Mg. of (dl)-2-(6,11-dihydrodibenzo-[b.e.]-thiepin-11-one-3-yl) propionic acid S-oxide in five ml. of methanol is titrated with 1N methanolic potassium hydroxide to a faint orange color, followed by discharging the color by the addition of three mg. of solid (dl)-2-(6,11-dihydrodibenzo-[b.e.]-thiepin-11-one-3-yl) propionic acid S-oxide, yielding a solution containing potasium (dl)-2-(6,11-dihydrodibenzo-[b.e.]-thiepin-11-one-3-yl) propionate S-oxide. A solution of 40 mg. of calcium carbonate dissolved in the minimum amount of 1N hydrochloric acid necessary to effect solution of the calcium carbonate, is buffered with 100 mg. of solid ammonium chloride, followed by the further addition of five ml. of water. The thus obtained buffered calcium solution is then added to the solution of potassium (dl)-2-(6,11-dihydrodibenzo-[b.e.]-thiepin-11-one-3-yl) propionate S-oxide to yield a precipitate. The precipitate is collected, washed with water and dried at room temperature to yield calcium (dl)-2-(6,11-dihydrodibenzo-[b.e.]-thiepin-11-one-3-yl) propionate S-oxide.

Likewise magnesium (dl)-2-(6,11-dihydrodibenzo-[b.e.]-thiepin-11-one-3-yl) propionate S-oxide is prepared by substituting magnesium carbonate for calcium carbonate.

In like manner the calcium and magnesium salts of

(dl)-2-(8-chloro-6,11-dihydrodibenzo-[b.e.]-thiepin-11-one-3-yl) propionic acid S-oxide,

(dl)-2-(8-methoxy-6,11-dihydrodibenzo-[b.e.]-thiepin-11-one-3-yl) propionic acid S-oxide

(dl)-2-(8-chloro-6,11-dihydrodibenzo-[b.e.]-thiepin-11-one-3-yl) propionic acid, and

(dl)-2-(8-methoxy-6,11-dihydrodibenzo-[b.e.]-thiepin-11-one-3-yl) propionic acid, are prepared

EXAMPLE 14

200 Mg. of 6,11-dihydrodibenzo-[b.e.]-thiepin-11-one-3-acetic acid S-oxide in one ml. of methanol is titrated with 1N methanolic potassium hydroxide to a faint orange color. A small amount of solid 6,11-dihydrodibenzo-[b.e.]-thiepin-11-one-3-acetic acid S-oxide was added to decolorize the solution. The solvent is stripped and the residue is dissolved in five ml. of water. The thus obtained aqueous solution of potassium 6,11-dihydrodibenzo-[b.e.]-thiepin-one-3-acetate S-oxide is added to a solution of 150 mg. of cupric nitrate trihydrate in five ml. of water. The precipitate which forms is collected, washed with water and dried in air to yield copper 6,11-dihydrodibenzo-[b.e.]-thiepin-11-one-3-acetate S-oxide.

In like manner the copper salts of

8-chloro-6,11-hydydrodibenzo-[b.e.]-thiepin-11-one-3-acetic acid S-oxide,

8-methoxy-6,11-hydydrodibenzo-[b.e.]-thiepin-11-one-3-acetic acid S-oxide,

8-chloro-6,11-dihydrodibenzo-[b.e.]-thiepin-11-one-3-acetic acid, and

8-methoxy-6,11-dihydrodibenzo-[b.e.]-thiepin-11-one-3-acetic acid, are prepared.

EXAMPLE 15

200 Mg. of (dl)-2-(6,11-dihydrodibenzo-[b.e.]-thiepin-11-one-3-yl) propionic acid S-oxide in one ml. of methanol is titrated with 1N methanolic potassium hydroxide to a faint orange color. A small amount of solid (dl)-2-(6,11-dihydrodibenzo-[b.e.]-thiepin-11-one-3-yl) propionic acid S-oxide is added to decolorize the solution. The solvent is stripped and the residue is dissolved in five ml. of water. The thus

obtained aqueous solution of potassium (dl)-2-(6,11-dihydrodibenzo-[b.e.]-thiepin-11-one-3-yl) propionate S-oxide is added to a solution of 150 mg.. of cupric nitrate trihydrate in five ml. of water. The precipitate which forms is collected, washed with water and dried in air to yield copper (dl)-2-(6,11-dihydrodibenzo-[b.e.]-thiepin-11-one-3-yl) propionate S-oxide.

In like manner the copper salts of

(dl)-2-(8-chloro-6,11-dihydrodibenzo-[b.e.]-thiepin-11-one-3-yl) propionic acid S-oxide,

(dl)-2-(8-methoxy-6,11-dihydrodibenzo-[b.e.]-thiepin-11-one-3-yl) propionic acid S-oxide,

(dl)-2-(8-chloro-6,11-dihydrodibenzo-]b.e.]-thiepin-11-one-3-yl) propionic acid, and

(dl)-2-(8-methoxy-6,11-dihydrodibenzo-]b.e.]-thiepin-11-one-3-yl) propionic acid, are prepared.

EXAMPLE 16

200 Mg. of 6,11-dihydrodibenzo-[b.e.]-thiepin-11-one-3-acetic acid S-oxide in 15 ml. of hot benzene is treated with 60 mg. of isopropylamine. The solution is allowed to cool to room temperature and the product is filtered off, washed with ether and dried to yield isopropylammonium 6,11-dihydrodibenzo-[b.e.]-thiepin-11-one-3-acetate S-oxide.

Likewise other salts, e.g., amine salts, such as, diethylamine, ethanolamine, piperidine, tromethamine, choline, and caffeine, of 6,11-dihydrodibenzo-[b.e.]-thiepin-11-one-3-acetic acid S-oxide are prepared by substituting each of the respective amines for isopropylamine.

In like manner the isopropylammonium and other amine salts of

8-chloro-6,11-dihydrodibenzo-[b.e.]-thiepin-11-one-3-acetic acid S-oxide,

8-methoxy-6,11-dihydrodibenzo-[b.e.]-thiepin-11-one-3-acetic acid S-oxide,

8-chloro-6,11-dihydrodibenzo-[b.e.]-thiepin-11-one-3-acetic acid, and

8-methoxy-6,11-dihydrodibenzo-[b.e.]-thiepin-11-one-3-acetic acid, are prepared.

EXAMPLE 17

193 Mg. of (dl)-2-(6,11-dihydrodibenzo-[b.e.]-thiepin-11-one-3-yl) propionic acid S-oxide in ten ml. of benzene is treated with 60 mg. of piperidine. The solution obtained is allowed to stand for one hour and the crystalline material which forms is filtered, washed with ether and air dried to yield piperidinium (dl)-2-(6,11-dihydrodibenzo-[b.e.]-thiepin-11-one-3-yl) propionate S-oxide.

Likewise other salts, e.g., amine salts, e.g., isopropylamine, diethylamine, ethanolamine, tromethamine, choline, and caffeine, of (dl)-2-(6-,11-dihydrodibenzo-[b.e.]-thiepin-11-one-3-yl) propionate S-oxide are prepared by substituting each of the respective amines for piperidine.

In like manner the piperidinium and other amine salts of

(dl)-2-(8-chloro-6,11-dihydrodibenzo-[b.e.]-thiepin-11-one-3-yl) propionic acid S-oxide,

(dl)-2-(8-methoxy-6,11-dihydrodibenzo-[b.e.]-thiepin 11-one-3-yl) propionic acid S-oxide,

(dl)-2-(8-chloro-6,11-dihydrodibenzo-[b.e.]-thiepin-11-one-3-yl) propionic acid, and

(dl)-2-(8-methoxy-6,11-dihydrodibenzo-[b.e.]-thiepin 11-one-3-yl) propionic acid, are prepared.

EXAMPLE 18

One g. of potassium 6,11-dihydrodibenzo-[b.e.]-thiepin-11-one-3-acetate S-oxide is dissolved in 50 ml. of water and the solution is acidified with 20 ml. of 3N aqueous hydrochloric acid. The reaction mixture is extracted twice with ethyl acetate (25 ml. portions) and the extracts are combined, washed with 50 ml. of water and dried over magnesium sulfate. The solvent is evaporated under reduced pressure and the residue is recrystallized from benzene to yield 6,11-dihydrodibenzo-[b.e.]-thiepin-11-one-3-acetic acid S-oxide.

Similarly, other salts, e.g., sodium, ammonium, calcium, amine, and the like, of 6,11-dihydrodibenzo-[b.e.]-thiepin-11-one-3-acetic acid S-oxide are converted to 6,11-dihydrodibenzo-[b.e.]-thiepin-11-one-3-acetic acid.

In like manner, substituting the salts, e.g., potassium, sodium, ammonium, calcium, amine, and the like, of

8-chloro-6,11-dihydrodibenzo-[b.e.]-thiepin-11-one-3-acetic acid S-oxide,

8-methoxy-6,11-dihydrodibenzo-[b.e.]-thiepin-11-one-3-acetic acid S-oxide,

8-chloro-6,11-dihydrodibenzo-[b.e.]-thiepin-11-one-3-acetic acid,

8-methoxy-6,11-dihydrodibenzo-[b.e.]-thiepin-11-one-3-acetic acid,

(dl)-2-(6,11-dihydrodibenzo-[b.e.]-thiepin-11-one-3-yl) propionic acid S-oxide,

(dl)-2-(8-chloro-6,11-dihydrodibenzo-[b.e.]-thiepin-11-one-3-yl) propionic acid S-oxide,

(dl)-2-(8-methoxy-6,11-dihydrodibenzo-[b.e.]-thiepin-11-one-3-yl) propionic acid S-oxide,

(dl)-2-(8-chloro-6,11-dihydrodibenzo-[b.e.]-thiepin-11-one-3-yl) propionic acid, and,

(dl)-2-(8-methoxy-6,11-dihydrodibenzo-[b.e.]-thiepin-11-one-3-yl) propionic acid, is productive of the corresponding free acids.

EXAMPLE 19

One g. of methyl 6,11-dihydrodibenzo-[b.e.]-thiepin-11-one-3-acetate S-oxide is dissolved in 500 ml. of toluene containing 5 g. of n-octanol and 0.5 g. of p-toluenesulfonic acid. The reaction mixture is heated in a nitrogen atmosphere and a total of 350 ml. of toluene is slowly distilled out over a period of five hours. The reaction mixture is cooled and concentrated to about 10 ml. by evaporation under reduced pressure. The residue is then chromatographed on 200 g. of silica gel eluting with hexane:ethyl acetate (1:8) to yield octyl 6,11-dihydrodibenzo-[b.e.]-thiepin-11-one-3-acetate S-oxide.

Similarly, other lower esters (e.g., the propyl ester) of 6,11-dihydrodibenzo-[b.e.]-thiepin-11-one-3-acetate acid S-oxide can be transesterified to a higher ester (e.g., the decyl ester) of 6,11-dihydrodibenzo-[b.e.]-thiepin-11-one-3-acetic acid S-oxide.

In like the other lower esters of the acids described herein can be transesterified to higher esters.

EXAMPLE 20

Five hundred mg. of dodecyl 6,11-dihydrodibenzo-[b.e.]-thiepin-11-one-3-acetate S-oxide is refluxed with 250 ml. of absolute ethanol containing 10 mg. of sodium cyanide for 18 hours in a nitrogen atmosphere. The reaction mixture is cooled and evaporated under reduced pressure to yield a residue which is chromatographed on 100 g. of silica gel eluting with hexane:ethyl acetate (1:8) to yield ethyl 6,11-dihydrodibenzo-[b.e.]-thiepin-11-one-3-acetate S-oxide.

Similarly, other higher esters (e.g., the nonyl ester) of 6,11-dihydrodibenzo-[b.e.]-thiepin-11-one-3-acetic acid S-oxide can be transesterified to a lower ester (e.g., the hexyl ester) of 6,11-dihydrodibenzo-[b.e.]-thiepin-11-one-3-acetic acid S-oxide.

In like manner other higher esters of the acids described herein can be transesterified to lower esters.

EXAMPLE 21

| Ingredients | Quantity per tablet, mgs. |
|---|---|
| 6,11-dihydrodibenzo-[b.e.]-thiepin-11-one-3-acetic acid S-oxide | 150 |
| cornstarch | 40 |
| sucrose | 200 |

The above ingredients are thoroughly mixed and pressed into single scored tablets.

EXAMPLE 22

| Ingredients | Quantity per tablet, mgs. |
|---|---|
| (dl)-2-(6,11-dihydrodibenzo-[b.e.]-thiepin-11-one-3-yl) propionic acid S-oxide | 25 |
| cornstarch | 100 |
| magnesium stearate | 2 |

The above ingredients are mixed intimately and passed into single scored tablets.

EXAMPLE 23

| Ingredients | Quantity per tablet, mgs. |
|---|---|
| potassium 6,11-dihydrodibenzo-[b.e.]-thiepin-11-one-3-acetate S-oxide | 150 |
| lactose | 190 |

The above ingredients are mixed and introduced into a hard-shell gelatin capsule.

EXAMPLE 24

| Ingredients | Quantity per tablet, mgs. |
|---|---|
| calcium 6,11-dihydrodibenzo-[b.e.]-thiepin-11-one-3-acetate S-oxide | 150 |
| lactose | 182 |
| magnesium stearate | 8 |

The above ingredients are mixed and introduced into a hard-shell gelatin capsule.

EXAMPLE 25

| Ingredients | Quantity per tablet, mgs. |
|---|---|
| isopropylammonium 6,11-dihydro dibenzo-[b.e.]-thiepin-11-one-3-acetate S-oxide | 150 |
| cornstarch | 100 |
| lactose | 370 |
| magnesium stearate | 2 |

The above ingredients are mixed intimately and pressed into single scored tablets.

EXAMPLE 26

| Ingredients | Quantity per tablet, mgs. |
|---|---|
| methyl 6,11-dihydrodibenzo-[b.e.]-thiepin-11-one-3-acetate S-oxide | 25 |
| lactose | 225 |

The above ingredients are mixed and introduced into a No. 1 hard-shell gelatin capsule.

EXAMPLE 27

| Ingredients | Quantity per tablet, mgs. |
|---|---|
| 6,11-dihydrodibenzo-[b.e.]-thiepin-11-one-3-acetic acid S-oxide | 150 |
| sucrose | 245 |

The above ingredients are thoroughly mixed and processed into single scored tablets, one tablet being administered every three to four hours.

EXAMPLE 28

| Ingredients | Quantity per tablet, mgs. |
|---|---|
| isoamyl 6,11-dihydrodibenzo-[b.e.]-thiepin-11-one-3-acetate S-oxide | 200 |
| cornstarch | 100 |
| lactose | 368 |
| magnesium stearate | 2 |

The above ingredients are mixed intimately and pressed into single scored tablets.

EXAMPLE 29

| Ingredients | Quantity per tablet, mgs. |
|---|---|
| (dl)-2-(6,11-dihydrodibenzo-[b.e.]-thiepin-11-one-3-yl propionic acid S-oxide | 25 |
| lactose | 225 |
| detrose | 10 |

The above ingredients are mixed and introduced into a hard-shell gelatin capsule.

EXAMPLE 30

| Ingredients | Quantity per tablet, mgs. |
|---|---|
| methyl 6,11-dihydrodibenzo-[b.e.]-thiepin-11-one-3-acetate S-oxide | 150 |
| lactose | 99 |

The above ingredients are mixed and introduced into a hard-shell gelatin capsule.

EXAMPLE 31

| Ingredients | Quantity per tablet, mgs |
|---|---|
| isoamyl 6,11-dihydrodibenzo-[b.e.]-thiepin-11-one-3-acetate S-oxide | 200 |
| lactose | 135 |
| magnesium stearate | 5 |

The above ingredients are mixed and pressed into single tablets.

## EXAMPLE 32

| Ingredients | Quantity per tablet, mgs. |
| --- | --- |
| calcium 6,11-dihydrodibenzo-[b.e.]-thiepin-11-one-3-acetate S-oxide | 150 |
| cornstarch (paste) | 50 |
| magnesium stearate | 0.8 |
| lactose | to 500 |

The above ingredients are thoroughly mixed and pressed into single scored tablets.

## EXAMPLE 33

| Ingredients | Quantity per tablet, mgs. |
| --- | --- |
| potassium 6,11-dihydrodibenzo-[b.e.]-thiepin-11-one-3-acetate S-oxide | 125 |
| cornstarch | 38 |
| magnesium stearate | 0.76 |
| polyvinylpyrrolidone | 17) |
| lactose | to 380 |

The above ingredients are mixed intimately and pressed into single scored tablets.

## EXAMPLE 34

| Ingredients | Quantity per tablet, mgs. |
| --- | --- |
| isopropylammonium 6,11-dihydrodi benzo-[b.e.]-thiepin-11-one-3-acetate S-oxide | 250 |
| cornstarch | 38 |
| lactose | to 380 |

The above ingredients are mixed and introduced into a hard-shell gelatin capsule.

EXAMPLE 35

| Ingredients | Quantity per tablet, mgs. |
|---|---|
| isoamyl 6,11-dihydrodibenzo-[b.e.]-thiepin-11-one-3-acetate S-oxide | 350 |
| lactose | 72 |
| magnesium stearate | 8 |

The above ingredients are mixed and introduced into a hard-shell gelatin capsule.

EXAMPLE 36

An injectable preparation buffered to a pH of 8.5 is prepared having the following composition:

| | |
|---|---|
| (dl)-2-(6,11-dihydrodibenzo-[b.e.]-thiepin-11-one-3-yl) propionic acid S-oxide | 0.2 g |
| $K_2HPO_4$ buffer (0.4 M. solution) | 2 ml. |
| KOH (1N) | 8.6 ml. |
| water (sterile) | to 20 ml. |

EXAMPLE 37

A suppository totaling 2.8 grams is prepared having the following composition:

| | |
|---|---|
| (dl)-2-(6,11-dihydrodibenzo-[b.e.]-thiepin-11-one-3-yl) propionic acid S-oxide | 25 mg. |
| Witepsol H-15 (triglycerides of saturated vegetable fatty acids; a product of Riches-Nelson, Inc., New York, N.Y.) | balance |

EXAMPLE 38

An oral suspension for pediatric use is prepared having the following composition:

| | |
|---|---|
| (dl)-2-(6,11-dihydrodibenzo-[b.e.]-thiepin-11-one-3-yl) propionic acid S-oxide | 0.25 g. |
| fumaric acid | 0.5 g |
| sodium chloride | 2.0 g |
| methyl paraben | 0.1 g. |
| granulated sugar | 25.5 g. |
| sorbitol (70% solution) | 12.85 g. |
| Veegum K (Vanderbilt Co.) | 1.0 g. |
| flavoring | 0.035 ml. |
| colorings | 0.5 mg. |
| distilled water | to 100 ml. |

EXAMPLES 39-40

Powdered top dressings for veterinary use are prepared having the following compositions:

| | Ex. 39 | Ex. 40 |
|---|---|---|
| (dl)-2-(6,11-dihydrodibenzo-[b.e.]-thiepin-11-one-3-yl) propionic acid S-oxide | 0.2 g. | 0.4 g. |
| sucrose | 5.7 g. | 3.7 g. |
| polyvinyl pyrrolidone | 0.3 g. | 0.3 g. |

EXAMPLE 41

A suppository totaling 2.8 grams is prepared having the following composition:

| | |
|---|---|
| 6,11-dihydrodibenzo-[b.e.]-thiepin-11-one-3-acetic acid S-oxide | 275 mg. |
| Witepsol H-15 | balance |

EXAMPLE 42

A) Heat Induced Dermal Trauma Assay

Test Animal: Albino rats, 120-140 g.

Procedure: The ventral area of the rats is shaved and depilated with a commercially available depilatory on the afternoon of the day prior to trauma. (The depilatory is applied for 15 minutes and then removed by washing with water.) The following day, a 25 mm diameter circle is traced on the abdomen beneath the sternum with a colored pen. A brass stamp of the same size, through which hot water (53°C) is circulated, is firmly applied to the center of the colored outline for 45 seconds while the rats are lightly anesthetized with ether. The test materials in ethanol are administered topically to the tramatized area immediately after withdrawing the stamp. The animals are sacrificed 3 hours later, at which time the ventral skin is removed and a piece of skin form the center of the marked area is punched out with a No. 10 cork bore and weighed.

End Point: The weight in mg of the inflammed piece of skin which is removed from the marked area with the cork bore.

The data obtained from the tests which were conducted to evaluate the compounds are as shown in the following table:

TABLE I

| Test Material | Dose mg. | Route | No. of Rats | Mean Body Wt. g.± S.E. | Time of Treatment Post Burn | Mean Disc. Wt. mg.± S.E. |
|---|---|---|---|---|---|---|
| Control | - | Top | 6 | 138±2 | 0 | 500.7±13.6 |
| 8-chloro-6,11,di- | 0.006 | Top | 6 | 140±1 | 0 | 296.8±21.8 |
| hydrodibenzo- | 0.025 | Top | 6 | 130±2 | 0 | 218.8±6.5 |
| [b.e.]-thiepin- | 0.1 | Top | 6 | 132±3 | 0 | 201.4±12.0 |
| 3-acetic acid | 0.4 | Top | 6 | 133±4 | 0 | 147.2±10.2 |
| 8-methoxy-6,11- | 0.006 | Top | 6 | 132±4.5[b] | 0 | 279.6±13.17 |
| dihydrodibenzo- | 0.025 | Top | 6 | 134±2.3[a] | 0 | 241.6±7.56 |
| [b.e.]-thiepin- | 0.1 | Top | 6 | 133±2.8[a] | 0 | 197.5±25.07 |
| 3-acetic acid | 0.4 | Top | 6 | 141±2.2[a] | 0 | 177.9±18.98 |

[a] $p < .01$
[b] $p < .05$

B) Mouse Acute Oral Toxicity ($LD_{50}$)

Protocol: The test material is suspended in an aqueous vehicle containing carboxymethyl cellulose. A volume of 10 ml/kg body weight is used for each dose. The test material is given orally by stomach tube to 24 male Swiss-Webster mice, 6 per group, on the first day of the test, using doses of 250, 500, 1000 and 2000 mg./kg. body weight of test compound. Another group of 6 mice, receiving the vehicle only, serve as the control. The mice are observed for deaths over a 14-day period.

Using the above protocol, the acute oral $LD_{50}$ of 8-chloro-6,11-dihydrodibenzo-[b.e.]-thiepin-3-acetic acid is approximately 300mg/kg and of 6,11-dihydrodibenzo-[b.e.]-thiepin-3-acetic acid S-oxide is $>$2000 mg/kg.

CLAIMS:

1. A compound selected from those having the formula:

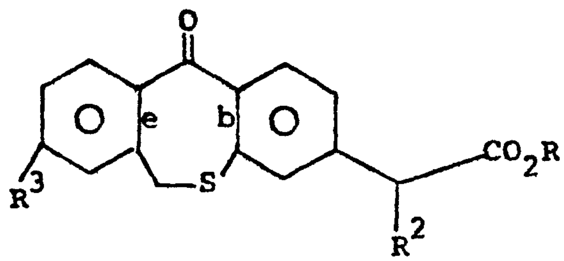

(II)

wherein R is hydrogen, an alkyl group containing from one to twelve carbon atoms, or a pharmaceutically acceptable salt thereof when R is hydrogen, $R^2$ is hydrogen or methyl and $R^3$ is chloro or methoxy.

2. The compound of Claim 1 wherein R and $R^2$ are hydrogen and $R^3$ is chloro, 8-chloro-6,11-dihydrodibenzo-[b.e.]-thiepin-11-one-3-acetic acid.

3. The compound of Claim 1 wherein R and $R^2$ are hydrogen and $R^3$ is methoxy, 8-methoxy-6,11-dihydrodibenzo-[b.e.]-thiepin-11-one-3-acetic acid.

4. A pharmaceutical composition comprising a pharmaceutically acceptable non-toxic excipient and a therapeutically effective amount of a compound of claim 1.

5. A compound of claim 1 for use in a method of treating mammals.

6. Process for the production of 8-$R^3$ substituted-6,11-dihydrodibenzo-[b.e.]-thiepin-11-one compounds of claim 1, which comprises one or more of the following steps:

(a) treating 3-diazoacetyl-8-$R^3$ substituted-6,11-dihydrodibenzo-[b.e.]-thiepin-11-one in the

presence of a $C_1$-$C_{12}$ alkanol to obtain the corresponding ester of (dl)-2- (8-$R^3$ substituted-6,11-dihydrodibenzo-[b.e.]-thiepin-11-one-3-yl) propionic acid;

(b) hydrolyzing a $C_1$-$C_{12}$ alkyl ester of 8-$R^3$ substituted-6,11-dihydrodibenzo-[b.e.]-thiepin-11-one-3-acetic acid to obtain 8-$R^3$ substituted-6,11-dihydrodibenzo-[b.e.]-thiepin-11-one-3-acetic acid;

(c) esterifying an acid of Formula (II) to obtain the corresponding $C_1$-$C_{12}$ alkyl esters;

(d) converting an acid of Formula (II) to pharmaceutically acceptable salts thereof;

(e) hydrolyzing an ester of Formula (II) to obtain the corresponding free acid or salt thereof;

(f) transesterifying a lower ester of Formula (II) to obtain a higher ester thereof;

(g) transesterifying a higher ester of Formula (II) to obtain a lower ester thereof;

(h) converting a salt of Formula (II) to the corresponding free acid;

(i) converting a salt of Formula (II) to another salt by a salt interchange.

7. A compound selected from those having the formula:

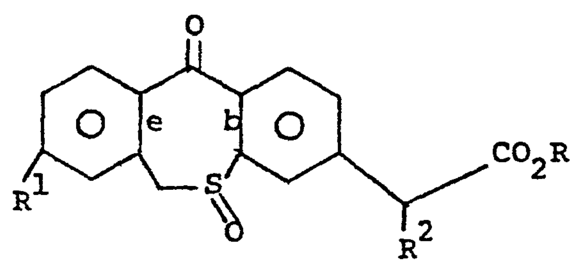

(I)

wherein R is hydrogen, an alkyl group containing from one to twelve carbon atoms, or a pharmaceutically acceptable salt thereof when R is hydrogen, $R^1$ is hydrogen, chloro or methoxy and $R^2$ is hydrogen or methyl

8. A pharmaceutical composition comprising a pharmaceutically acceptable non-toxic excipient and a therapeutically effective amount of a compound of claim 7.

9. A compound of claim 7 for use in a method of treating mammals.

10. Process for the production of 6,11-dihydrodibenzo-[b.e.]-thiepin-11-one S-oxides of claim 7, which comprises one or more of the following steps:

(a) oxidizing a non-S-oxide free acid or ester, otherwise corresponding to a compound of Formula (I), to obtain a compound of Formula (I);

(b) hydrolyzing a $C_1$-$C_{12}$ alkyl ester of Formula (I) to obtain the corresponding free acid of Formula (I) or salt thereof;

(c) esterifying a free acid of Formula (I) to obtain the corresponding $C_1$-$C_{12}$ alkyl esters;

(d) converting an acid of Formula (I) to a pharmaceutically acceptable salt thereof;

(e) hydrolyzing an ester of Formula (I) to obtain the corresponding free acid;

(f) transesterifying a lower ester of Formula (I) to obtain a higher ester thereof;

(g) transesterifying a higher ester of Formula (I) to obtain a lower ester thereof;

(h) converting the salts of Formula (I) to the corresponding free acid;

(i) converting a salt of Formula (I) to another salt by a salt interchange.